# FASCICULE DE BREVET EUROPEEN

(11) **EP 2 600 845 B1**
(45) Date de publication et mention de la délivrance du brevet: **14.05.2014**
(21) Numéro de dépôt: 11738751.4
(22) Date de dépôt: 03.08.2011
(51) Int. Cl.: A61K 9/20, A61K 36/889, A61K 47/10, A61P 13/00, A61P 13/08

(54) **COMPOSITION PHARMACEUTIQUE CONTENANT UN EXTRAIT DE SERENOA REPENS**
PHARMAZEUTISCHE ZUSAMMENSETZUNG MIT EINEM EXTRAKT AUS SERENOA REPENS
PHARMACEUTICAL COMPOSITION CONTAINING A SERENOA REPENS EXTRACT

(30) Priorité: 03.08.2010 FR 1056408
(43) Date de publication de la demande: 12.06.2013
(73) Titulaire: Pierre Fabre Médicament, 92100 Boulogne-Billancourt (FR)
(72) Inventeur: LEVERD, Elie, F-81100 Castres (FR); BOUGARET, Joël, F-31460 Francarville (FR)
(74) Mandataire: Regimbeau
(86) Numéro de dépôt international: PCT/EP2011/063395
(87) Numéro de publication internationale: WO 2012/017022

(56) Documents cités:
- EP-A2- 0 265 338
- WO-A1-01/00038
- WO-A1-99/20289
- WO-A2-2009/117151
- FR-A1- 2 791 573
- US-A1- 2003 096 009
- US-A1- 2006 121 129
- Anonymous: "PROSTASERENE Weichkapseln", , 1 octobre 2009 (2009-10-01), pages 1-5, XP002618806, Extrait de l'Internet: URL:http://webcache.googleusercontent.com/ search?hl=de&q=cache:gKIY328tDJQJ:http://1 95.130.154.23/urlnotices/lid/lid.aspx?sLID =8118&sUID=QVSRUGUBBN+PROSTASERENE+Weichka pseln&ct=clnk [extrait le 2011-02-07]
- COLE ET AL: "Challenges and opportunities in the encapsulation of liquid and semi-solid formulations into capsules for oral administration", ADVANCED DRUG DELIVERY REVIEWS, vol. 60, no. 6, 21 décembre 2007 (2007-12-21), pages 747-756, XP022476862, ELSEVIER BV, AMSTERDAM, NL ISSN: 0169-409X, DOI: 10.1016/J.ADDR.2007.09.009

## Description

La présente invention a pour objet une composition contenant un extrait de Serenoa repens, aussi appelé Saw palmetto, par exemple un extrait lipido stérolique de Serenoa repens, associé à un polyéthylène glycol ou macrogol de haut poids moléculaire, solide à température ambiante.

Le Serenoa repens est connu pour son activité dans le traitement des désordres prostatiques, notamment dans le traitement et la prévention de l'hyperplasie bénigne de la prostate et/ou du cancer de la prostate. Il entre aussi dans la composition de nombreux compléments nutritionnels.

US 2006/0121129 décrit un complément alimentaire pour le traitement ou la prévention des maladies de la prostate contenant du Saw palmetto et d'autres composés comme par exemple des tocophérols, du sélénium, du lycopène, du zinc, de l'acide folique, entre autres. La composition selon US2006/0121129 peut contenir une quantité de 45 à 86 % de Saw palmetto et en particulier représente 60 à 80% des composants actifs de la composition. Une composition contenant entre autres les composés cités précédemment peut contenir 320 mg de Saw palmetto dans des comprimés oblongs. Les exemples décrivent des comprimés contenant les différents ingrédients actifs dont 320 mg de Saw palmetto et parmi les ingrédients d'enrobage la présence de polyéthylène glycol. Toutefois la teneur en polyéthylène glycol et son poids moléculaire moyen ne sont pas indiqués. En outre le polyéthylène glycol a uniquement la fonction d'agent d'enrobage dans ces compositions.

La demande de brevet internationale WO 99/20289 décrit des procédés visant à encapsuler des concentrés d'extraits végétaux dans des capsules de gélatine souples grâce à l'utilisation d'un liquide de remplissage compatible avec la nature de l'extrait végétal. Ainsi, pour tout extrait végétal hydrophobe, ce document recommande l'utilisation d'un liquide de remplissage hydrophobe. Dans le cas particulier d'ELSSR (extrait végétal hydrophobe), WO 99/20289 incite donc à utiliser un liquide de remplissage hydrophobe tel que l'huile de soja (p9, 128-30, exemples 2 et 3 p13). Ce document détourne donc l'homme du métier de l'utilisation d'un liquide de remplissage hydrophile, tel que les PEG, pour l'encapsulation d'ELSSR.

WO2009/117151 décrit la formulation et la fabrication de compléments alimentaires contenant des composés actifs non polaires sous forme de poudre à dissoudre dans l'eau. Les PEG utilisés dans cette formulation jouent le rôle de surfactants et sont dérivés de la vitamine E (tocophérol ou tocotriénol), tels que les TPGD, TPGS et leurs analogues (p 27121 à p 32 130 et p 91 114 à 27)).

WO2009/103477 décrit des compositions pour le traitement de l'hypertrophie bénigne de la prostate et du cancer de la prostate. Les compositions peuvent être des gélules contenant de l'extrait lipido-stérolique de Serenoa repens, et de Echinacea angustifolia et d'Hyperricum perforatum avec de la methylselenocysteine.

La demande de brevet FR 2 791 573 décrit l'utilisation d'un extrait de Serenoa repens pour la fabrication d'un médicament destiné au traitement du cancer de la prostate. Ledit extrait lipido-stérolique peut être utilisé sous diverses formes de préparations pharmaceutiques, en particulier des gélules ou des capsules. De telles formulations ne sont pas détaillées.

La demande de brevet européen EP 0 265 338 décrit une composition pharmaceutique de remplissage stable comprenant en poids 10 à 40 % d'un extrait lipido-stérolique de Serenoa repens et 60 à 90 % d'un ou plusieurs polyéthylèneglycols (ou PEG) choisis parmi les PEG 6 000, PEG 10 000 et PEG 20 000 et est exemplifiée par une formule unitaire préférée contenant 160 mg d'extrait et 290 mg de polyoxyéthylène glycol 10 000. Cette composition peut être administrée par voie orale, en particulier sous la forme de capsule contenant la composition de remplissage. Cette demande de brevet enseigne les avantages de l'utilisation des proportions particulières précitées excipient/ principe actif, par exemple en terme de vitesse de libération du principe actif et d'absence de fuite entre corps et tête de la gélule, d'absence de démixtion et de fuite du principe actif. Cette demande de brevet ne suggère donc pas l'utilisation de proportions différentes et en particulier d'une quantité plus importante de principe actif que de PEG.

L'extrait lipido-stérolique de Serenoa repens est prescrit dans le traitement des troubles mictionnels modérés liés à une hypertrophie bénigne de la prostate. Il est commercialisé sous la marque Permixon® et se présente sous la forme de gélules de taille n°1 contenant 160 mg de l'extrait lipido stérolique de Serenoa repens et du PEG 10000.

La dose effective journalière est de 320 mg divisée en deux administrations quotidiennes. A l'heure actuelle, les patients doivent donc prendre deux gélules par jour de médicament préférentiellement au moment des repas. Il a été constaté que l'observance des patients à suivre ce traitement avec ce rythme d'administration n'était pas optimale.

Le but de la présente invention est donc de fournir aux patients ayant besoin de ce traitement, une composition pharmaceutique orale permettant l'administration quotidienne en une seule prise d'une dose effective de l'extrait lipido stérolique de Serenoa repens, par exemple une dose de 320 mg par prise dudit extrait de façon à améliorer l'observance du traitement.

Cependant il n'est pas envisageable de développer une composition de remplissage comprenant la dose journalière de 320 mg d'extrait lipido stérolique de Serenoa repens associée dans les mêmes proportions que dans la demande EP 0 265 338 A2 à l'excipient. En effet cela reviendrait à doubler la quantité d'excipient et donc à ajouter aux 320 mg d'extrait lipido stérolique de Serenoa repens par exemple 580 mg de PEG 10 000, sous une présentation unitaire destinée à la voie orale. Cela nécessiterait l'utilisation, pour contenir la composition de remplissage, de gélules de taille 00 (volume 0,91 ml) ou 000 (volume 1,34 ml) par exemple. Devant ce type de gélules, certains patients sont dissuadés de suivre le traitement car ils ressentent une appréhension au vu de la taille de la gélule. D'autres patients vont éprouver des difficultés ou ne pas pouvoir avaler ces gélules comme cela peut-être le cas, chez une population de patients plus âgés par exemple. L'amélioration de l'observance des patients à suivre ce traitement grâce à une formulation à prise unique journalière n'est donc pas garantie si les patients sont découragés de prendre le traitement pour une autre raison comme par exemple l'appréhension de la prise du traitement liée à la taille de la gélule, et/ou la difficulté d'avaler l'unique dose journalière.

La présente invention propose donc de résoudre ce problème. De façon totalement inattendue, les inventeurs ont montré qu'il était possible d'obtenir une composition de remplissage comprenant un extrait lipido stérolique de Serenoa repens (ELSSR) associé avec des polyéthylène glycols ou macrogols de haut poids moléculaire, dans des proportions différentes de celles décrites dans la demande de brevet EP 0 265 338, et ceci sans que cela n'affecte le remplissage des capsules en regard des deux étapes essentielles de la fabrication que sont la préparation du mélange ELSSR et polyethylene glycol, vrac, à chaud, et sa répartition et sans que cela n'affecte la cinétique de dissolution de la composition. Les rhéologies des mélanges vracs fondus selon la demande de brevet EP 0 265 338 et d'autre part selon la présente invention sont similaires comme le prouvent les études de viscosité et de caractère filant indiquées ci-après.

Cette composition de remplissage peut donc être par exemple utilisée pour remplir des capsules de taille 0 (volume 0,68 ml) permetant l'administration d'une dose unitaire d'ELSSR. Cette taille se situe, avec la taille 0 el (volume 0,78 ml), à la limite d'avalabilité et évite l'appréhension de la prise du traitement liée à la taille de la capsule, et/ou la difficulté d'avaler la capsule et donc améliore l'observance du malade au traitement.

La présente invention concerne donc une composition pharmaceutique sous forme d'une capsule contenant une composition de remplissage comprenant, en poids par rapport au poids total de la composition de remplissage :
- 50 à 90% d'un extrait de Serenoa repens, et
- 10 à 50 % d'au moins un polyéthylène glycol ou macrogol de haut poids moléculaire solide à température ambiante.

Dans un mode de réalisation, la présente invention porte sur une composition pharmaceutique sous forme d'une capsule contenant une composition de remplissage consistant en, en poids par rapport au poids total de la composition de remplissage :
- 50 à 90% d'un extrait de Serenoa repens, et
- 10 à 50 % d'au moins un polyéthylène glycol ou macrogol de haut poids moléculaire solide à température ambiante. Dans ce mode de réalisation la capsule ne contient rien d'autre que ladite composition de remplissage.

Par composition de remplissage selon l'invention on entend la composition qui sera contenue dans la capsule dans la composition pharmaceutique orale finale.

Par capsule selon l'invention on entend des capsules à enveloppe dure (ou gélule) ou molle. Préférentiellement, la capsule selon l'invention est une capsule à enveloppe dure. En effet ce type de capsule présente un grand nombre d'avantages comme par exemple l'absence de risque de fuite et une augmentation de la facilité d'absorption par le patient.

Lorsque la composition selon l'invention est sous forme de capsules à enveloppe dure ou molle, elles sont préférentiellement réalisées à base de gélatine ou de tout autre polymère que la gélatine, tel que à titre d'exemple non limitatif l'hydroxypropylméthylcellulose, par des méthodes bien connues de l'homme du métier.

Les capsules à enveloppe dure (gélules) ou molle selon l'invention sont avantageusement de taille 1, 0 (0,68 ml), ou 0 el (0,78 ml), préférentiellement de taille 0, ou 0 el, préférentiellement de taille 0.

La capsule selon l'invention est pour une administration par voie orale. La capsule est avalée directement par le patient telle quelle.

Préférentiellement, l'extrait de Serenoa repens selon l'invention est un extrait lipido-stérolique de Serenoa repens.

L'extrait lipido-stérolique de Serenoa repens est bien connu de l'homme du métier. Il a par exemple, la composition décrite dans la demande de brevet EP 0 265 338 (colonne 1, lignes 46 à 59). Il peut être obtenu par des procédés bien connus de l'homme du métier et en particulier tel que décrit dans la demande de brevet FR 2 480754. Le procédé décrit dans la demande de brevet FR 2 480754 peut également être mis en oeuvre sans adjonction d'antioxydant pendant l'extraction. L'extraction peut être conduite partiellement sous gaz inerte, le passage sous gaz inerte, par exemple l'azote, peut être réalisé à partir de la seconde extraction. Le solvant d'extraction utilisé est par exemple l'hexane. L'étape de traitement de l'huile avec noir décolorant n'est pas préférentiellement effectuée.

Les polyéthylènes glycol selon l'invention sont préférentiellement des polymères polyéthers linéaires fabriqués à partir de monomères d'éthylène glycol, non dérivés de la vitamine E. Par au moins un polyéthylène glycol ou macrogol de haut poids moléculaire solide à température ambiante selon l'invention on entend préférentiellement au moins un polyéthylène glycol ou macrogol de poids moléculaire moyen (PM) compris entre 6 000 et 35 000, par exemple un PM de 6 000, de 8 000, de 10 000, de 20 000 ou de 35 000 ou leur mélange. Avantageusement, dans la composition selon la présente invention, le PEG est choisi parmi les PEG de PM 6 000, 8 000, 10 000, 20 000 et 35 000 et leur mélange, de façon avantageuse les PEG de PM 10 000, 20 000 et 35 000 et leur mélange, de façon encore plus avantageuse les PEG de PM 10 000 et 20 000 et leur mélange. Préférentiellement, les compositions selon l'invention ne comprendront qu'un seul type de PEG de PM donné, par exemple un PEG de PM 10 000.

Dans un mode de réalisation, les polyéthylènes glycols utilisés pour la préparation de la composition selon la présente invention sont les polyéthylènes glycols de poids moléculaire moyen 6 000 et 8 000 : leurs plages de fusion sont respectivement comprises entre 55 °C - 63 °C et 60 °C - 63 °C et sont très peu différentes de celle du polyéthylène glycol 20 000 qui est comprise entre 60 °C - 63 °C (Handbook of Pharmaceutical Excipients 5th edition).

Selon la présente invention les pourcentages sont exprimés en pourcentage en poids (massique (m/m)) par rapport au poids total de la composition de remplissage.

Dans un mode de réalisation préféré, la composition de remplissage selon l'invention comprend, par rapport au poids total de la composition de remplissage, de 50 à 90 % (m /m), par exemple de 55 à 60%, par exemple de 60 à 90%, par exemple de 70 à 90 %, par exemple de 80 à 90 %, d'un extrait de Serenoa repens et de 10 % à 50 % (m /m), par exemple de 40 à 45%, par exemple de 10 à 40 %, par exemple de 10 à 30%, par exemple de 10 à 20%, d'au moins un polyéthylène glycol ou macrogol de haut poids moléculaire solide à température ambiante, préférentiellement de PM compris entre 6000 et 35000.

Dans un mode de réalisation, la composition de remplissage selon la présente invention comprend entre 55 et 60% d'extrait de Serenoa repens et entre 40 et 45% (m/m) d'au moins un polyéthylène glycol ou macrogol de haut poids moléculaire solide à température ambiante, préférentiellement de PM compris entre 6000 et 35000.

Selon un mode de réalisation de l'invention, la composition de remplissage comprend 58,2 % d'extrait lipido stérolique de Serenoa repens et 41,8 % d'au moins un PEG ou Macrogol de PM 6 000 à 35 000, par exemple, un PEG choisi parmi les PEG de PM 10 000, 20 000, et 35 000.

Dans un mode de réalisation, les pourcentages relatifs d'extrait de Serenoa repens et de PEG dans la composition de remplissage selon l'invention sont déterminés de manière à pouvoir obtenir des capsules dures donc de manière à ce que la composition de remplissage soit solide à température ambiante.

La composition selon l'invention peut être utilisée pour produire des doses d'administration unitaires comprenant chacune la quantité d'extrait de Serenoa repens que l'on veut administrer par jour à un patient.

Les compositions pharmaceutiques selon l'invention permettent préférentiellement l'administration orale quotidienne d'une quantité d'extrait de Serenoa repens supérieure à 180mg, préférentiellement supérieure à 250 mg, préférentiellement de 320mg en une seule dose (par exemple une capsule). Préférentiellement, les compositions pharmaceutiques selon l'invention comprennent donc plus de 180 mg, préférentiellement plus de 250 mg, encore préférentiellement autour ou de 320 mg d'extrait de Serenoa repens par unité (gélule, capsule etc...).

La présente invention a également pour objet un procédé de préparation de la composition selon la présente invention. Dans le procédé selon la présente invention, le mélange entre l'extrait lipido stérolique de Serenoa repens et le au moins un PEG est obtenu par co-fusion et la composition obtenue est conditionnée par remplissage dans des capsules. L'avantage de l'utilisation de PEG comme excipients de formulation de la composition de remplissage réside dans la facilité de mise en oeuvre du procédé de fabrication des capsules. En effet, l'ajout de PEG par co-fusion permet d'obtenir une composition de remplissage sous forme liquide à température modérée et solide à température ambiante.

Les capsules selon l'invention sont ainsi remplies à une température modérée où la composition de remplissage se présente sous forme liquide. Après refroidissement, la composition devient solide. Cela permet d'éviter les problèmes de démixtion et de fuite du principe actif hors de la capsule.

Lesdites capsules sont ensuite scellées. Le scellage peut être réalisé par banderolage ou par application d'un spray hydroalcoolique, par exemple sur la zone à sceller. Dans ce dernier cas, le spray hydroalcoolique est une solution pulvérisée d'un mélange d'alcool éthylique à 96 % et d'eau purifiée dont les proportions massiques sont respectivement comprises entre 50 - 55 % et 45 - 50 %, plus précisément égales à 52,9 % et 47,1 %.

Pour éviter toute fuite éventuelle d'ELSSR dans les formulations 320 mg, les capsules objets de l'invention sont scellées au niveau de leur interface tête/corps, soit par pulvérisation d'un spray hydroalcoolique, soit par dépôt d'une bande du polymère ayant servie à faire la capsule.

Le scellage par spray hydroalcoolique implique la pulvérisation d'une solution hydroalcoolique suivie d'un séchage par chauffage.

Le scellage par dépôt d'une bande de polymère est un banderolage, issu de l'application d'une solution de polymère suivie d'un séchage par chauffage.

La présente invention concerne en outre une composition pharmaceutique selon la présente invention pour son utilisation en tant que médicament.

Elle concerne en particulier une composition pharmaceutique selon la présente invention pour son utilisation dans le traitement et/ou la prévention des désordres prostatiques préférentiellement dans le traitement et/ou la prévention de l'hyperplasie bénigne de la prostate et/ou du cancer de la prostate et/ou de la prostatite et/ou des troubles associés tels que les troubles mictionnels liés à l'hypertrophie bénigne de la prostate et/ou au cancer de la prostate et/ou pour le traitement et/ou la prévention de l'incontinence urinaire

La composition selon la présente invention peut être administrée en association, de manière simultanée, séparée ou étalée dans le temps, avec une prostatectomie, une radiothérapie, et/ou une hormonothérapie, en vue du traitement ou de la prévention du cancer de la prostate comme indiqué dans la demande FR 2 791 573.

L'invention est illustrée par les exemples ci-dessous.

### Légende des figures :

Figure 1 : La figure 1 représente la viscosité (en Pa.s) à une température comprise entre 60°C et 65°C des compositions selon l'invention comparée à celle de la composition selon l'art antérieur (LT 812).
Figure 2 : La figure 2 représente la mesure de la force (g) de cohésion à une température comprise entre 60°C et 65°C des compositions selon l'invention comparée à celle de la composition selon l'art antérieur (LT812).
Figure 3 : La figure 3 représente la force (g) qui s'oppose à l'introduction de l'aiguille dans les compositions à une température de 25°C pour les compositions selon l'invention comparée à celle de la composition selon l'art antérieur (LT812).
Figure 4: La figure 4 représente la cinétique de dissolution d'une composition selon l'invention (LT 832) par rapport à celle de la composition selon l'art antérieur LT 828 (brevet EP 0265338).

### Exemple 1 : Compositions testées

### Préparation des compositions selon l'invention :

300 g des compositions centésimales tabulées ci-après ont été préparés par ajout sous agitation de l'ELSSR préalablement chauffé à 35 °C dans les macrogols fondus. Une partie des mélanges vrac est conservée pour les études rhéologiques, l'autre partie est répartie dans les gélules de taille 1 ou 0.

| | **LT812 Exemple comparatif** | **LT811** | **LT810** | **LT809** | **LT808** | **LT807** | **LT806** |
|---|---|---|---|---|---|---|---|
| **Taille capsule** | | 1 | 0 | 1 | 0 | 1 | 0 |
| **ELSSR** | 160 mg (35,6 %) | 320 mg (84,2 % ) | 320 mg (55,2 % ) | 320 mg (88,9 % ) | 320 mg (57,1 % ) | 320 mg (88,9 % ) | 320 mg (57,7 % ) |
| **Macrogol 10 000** | 290 mg (64,4 %) | | | | | 40 mg (11,1 % ) | 235 mg (42,3 % ) |
| **Macrogol 20 000** | | | | 40 mg (11,1 % ) | 240 mg (42,9 % ) | | |
| **Macrogol 35 000** | | 60 mg (15,8 %) | 260 mg (44,8 %) | | | | |

Le mélange LT812 correspond à la formule unitaire qui figure dans le brevet EP 0 265 338.

Les mélanges LT811, LT809 et LT807 sont répartis dans une gélule de taille 1, comme LT812, alors que les mélanges LT810, LT808 et LT806 le sont dans une gélule de taille 0.

### 1 - Viscosité :

La viscosité des mélanges fondus est déterminée à une température comprise entre 60°C et 65°C, à l'aide d'un viscosimètre cône-plateau HAAKE MK 500, avec une géométrie PK 1°, pour une vitesse de cisaillement de 123,4 s-1.

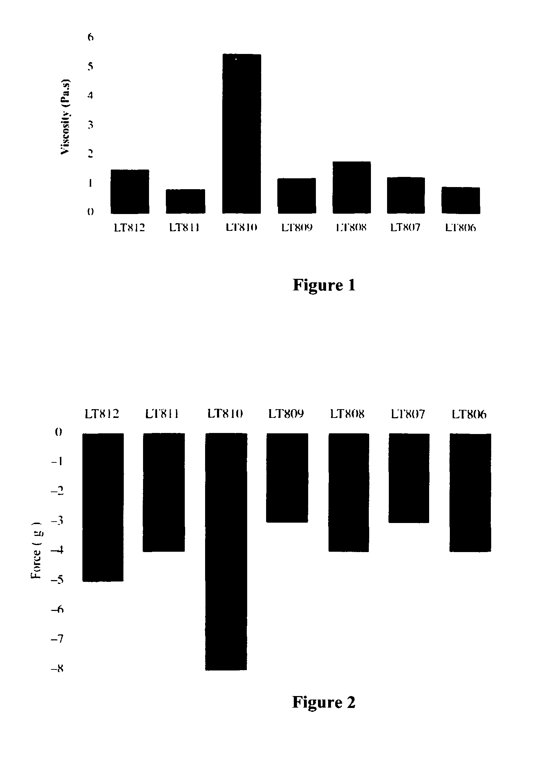

Les résultats sont illustrés dans la Figure 1 : Tous les mélanges présentent, à chaud, une viscosité voisine. La viscosité de la composition LT810: 320 mg ELSSR / 260 mg Macrogol 35 000 est légèrement différente mais n'empêche pas la mise en oeuvre de celle-ci.

Ce résultat indique de manière inattendue que le PEG de PM 35 000 qui n'était pas considéré dans le brevet EP 0265338 pour la préparation des gélules est adapté à la préparation des gélules contenant deux fois plus d'extrait lipido stérolique de Serenoa repens.

### 2 - Caractère Filant :

L'étude du caractère filant des mélanges fondus est conduite à une température comprise entre 60°C et 65°C, à l'aide d'un analyseur de texture STEVENS LFRA. Ce caractère filant est appréhendé lors du retrait d'un mobile cylindrique de diamètre 38,1 mm que l'analyseur de texture a fait pénétrer initialement dans le mélange fondu avec une vitesse de 4 mm/s sur une distance de 10 mm. Il est lié à la force de cohésion qui retient le mobile.

Les résultats sont illustrés dans la Figure 2 : comme déjà constaté à l'issue de l'étude de viscosité, la composition ELSSR : 320 mg / Macrogol 35 000 : 260 mg présente, à chaud, un comportement légèrement différent des autres compositions mais ce comportement reste acceptable.

La rhéologie des mélanges fondus ELSSR et polyéthylène glycols ou macrogols de haut poids moléculaire est appropriée pour leur préparation et leur répartition en gélules, même quand le pourcentage d'ELSSR présent est compris entre 50 % et 90 % (m/m).

### 3 - Pénétrométrie :

Par ailleurs, une étude de pénétromètrie a été effectuée sur ces mêmes mélanges, à une température de 25°C, pour appréhender leur consistance après l'étape de refroidissement qui suit la répartition en gélules.

L'étude est menée avec un analyseur de texture RHEO TA-XT2, équipé d'une aiguille cylindrique d'un diamètre de 2 mm qui pénètre dans le mélange avec une vitesse de 4 mm / s sur une distance de 10 mm. La force qui s'oppose à l'introduction de l'aiguille représente la consistance du mélange.

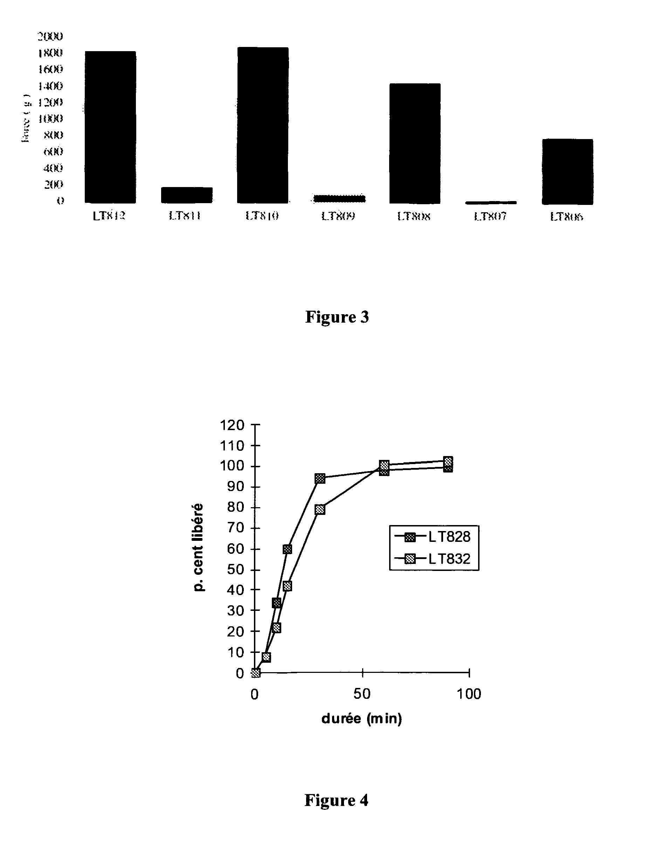

Les résultats sont illustrés dans la Figure 3 : ils indiquent que pour les formulations unitaires à 320 mg d'ELSSR, les plus fortes quantités de polyéthylène glycol ou macrogol permettent de s'approcher de la consistance de la formulation unitaire à 160 mg d'ELSSR. Cette consistance décroit avec le poids moléculaire moyen des polyéthylène glycols ou macrogols.

### 4 - Dissolution

Une étude de dissolution comparative d'une gélule de 320 mg d'ELSSR de composition selon la présente invention versus deux gélules de 160 mg d'ELSSR de composition selon l'art antérieur (EP0265338) a été conduite, basée sur le suivi de la libération de l'acide oléique.

Les caractéristiques des gélules sont tabulées ci-après :

| | Gélule 160 mg (non scellée) | Gélule 320 mg (scellée) |
|---|---|---|
| | Exemple comparatif | |
| ELSSR | 160 mg (35,6 %) | 320 mg (58,2 %) |
| Macrogol 10 000 | 290 mg (64,4 %) | 230 mg (41,8 %) |
| Taille de la gélule | 1 | 0 |
| Référence lot | LT828 | LT832 |

Les conditions opératoires de l'étude de dissolution sont les suivantes :
- appareil à palette tournante, vitesse : 120 tours / min
- milieu de dissolution : 1 litre de solution à 1 % (m/V) de laurylsulfate de sodium à 37 °C
- positionnement des gélules dans un panier métallique.

Les résultats de la Figure 4 démontrent l'équivalence des deux formulations en terme de dissolution bien que les formulations soient différentes et que la gélule soit plus grosse.

### 5 - Fabricabilité

La fabricabilité industrielle des nouvelles compositions a été vérifiée à travers la mise en oeuvre de 4 lots différents et l'application des deux techniques de scellage.

La conformité des lots préparés est confirmée sur les items suivant :
- dosage unitaire de l'ELSSR (320 mg +/- 5 %),
- uniformité du contenu par l'expression du coefficient de variation,
- profil de libération de l'acide oléique.

Les caractéristiques de ces lots et les résultats d'analyse sont tabulés ci-dessous :

| Référence du lot | SB0020B | SB0022E | SB0026A | SB0027A |
|---|---|---|---|---|
| Taille du lot | 198 300 gélules | 122 380 gélules | 137 580 gélules | 114 705 gélules |
| Scellage | Banderolage | Banderolage | Spray hydro-alc. | Spray hydro-alc. |
| Dosage ELSSR (304 à 336 mg) | 327,7 mg | 321,0 mg | 322,7 mg | 312,9 mg |
| Coef. variation | 2,2% | 1,6% | 0,6% | 0,9 % |
| Lib. ac. oléique (>80% en 60 mn | 93 % | 93 % | 97 % | 98 % |

En conclusion, la composition des gélules dosées à 320 mg d'ELSSR selon l'invention présente de manière inattendue compte tenu de la différence de formulation les mêmes propriétés rhéologiques que la composition des gélules dosées à 160 mg et les mêmes propriétés de cinétique de dissolution. Ceci est important pour la fabrication industrielle de cette composition et ses caractéristiques de biodisponibilité par voie orale.

La composition selon la présente invention permet de mettre à disposition des patients une forme galénique unitaire dosée à 320 mg d'ELSSR, qui correspond à la posologie quotidienne et favorise ainsi le respect du suivi du traitement.

### Exemple 2 : Autre composition selon l'invention

Une composition contenant les ingrédients suivants : ELSSR : 320 mg (90%), PEG 10 000 : 35,5mg (10%), a été préparée par ajout sans agitation de l'ELSSR préalablement chauffé à 35°C dans le macrogol fondu. Le mélange vrac est répartie dans les gélules de taille 1 scellées.

## Revendications

1. Composition pharmaceutique sous forme d'une capsule contenant une composition de remplissage comprenant, en poids par rapport au poids total de la composition de remplissage :
- 50 à 90% d'un extrait lipido stérolique de Serenoa repens, et
- 10 à 50 % d'au moins un polyéthylène glycol de poids moléculaire moyen compris entre 6000 et 35000.

2. Composition pharmaceutique selon la revendication 1, **caractérisée en ce que** la composition de remplissage comprend 55 à 60% de l'extrait lipido stérolique de Serenoa repens et 40 à 45% du au moins un polyéthylène glycol de poids moléculaire moyen compris entre 6 000 et 35 000.

3. Composition pharmaceutique selon la revendication 2, **caractérisée en ce que** la composition de remplissage comprend 58,2 % de l'extrait lipido stérolique de Serenoa repens et 41,8 % du au moins un polyéthylène glycol de poids moléculaire moyen compris entre 6 000 et 35 000.

4. Composition pharmaceutique selon l'une quelconque des revendications 1 à 3, **caractérisée en ce que** le au moins un polyéthylène glycol est choisi parmi le polyéthylène glycol de poids moléculaire moyen 10 000, le polyéthylène glycol de poids moléculaire moyen 20 000, et le polyéthylène glycol de poids moléculaire moyen 35 000.

5. Composition pharmaceutique selon l'une quelconque des revendications 1 à 4, **caractérisée en ce qu'**elle comprend 320mg de l'extrait lipido stérolique de Serenoa repens.

6. Composition pharmaceutique selon l'une quelconque des revendications 1 à 5, dans laquelle la capsule est une capsule à enveloppe dure.

7. Composition pharmaceutique selon l'une quelconque des revendications 1 à 6, **caractérisée en ce que** la capsule est de taille 1, 0 ou 0 el, préférentiellement de taille 0.

8. Procédé de préparation d'une composition pharmaceutique selon l'une quelconque des revendications 1 à 7, **caractérisée en ce que** l'on mélange l'extrait lipido stérolique de Serenoa repens avec le au moins un polyéthylène glycol par co-fusion et **en ce que** la composition obtenue est conditionnée par remplissage dans des capsules, lesdites capsules étant ensuite scellées.

9. Procédé selon la revendication 8, **caractérisé en ce que** le scellage est réalisé par banderolage ou pulvérisation d'un spray hydroalcoolique.

10. Composition pharmaceutique selon l'une quelconque des revendications 1 à 7, pour son utilisation en tant que médicament.

11. Composition pharmaceutique selon la revendication 10, pour son utilisation dans le traitement et/ou la prévention des désordres prostatiques préférentiellement dans le traitement et/ou la prévention de l'hyperplasie bénigne de la prostate et/ou du cancer de la prostate et/ou de la prostatite et/ou des troubles associés tels que les troubles mictionnels liés à l'hypertrophie bénigne de la prostate et/ou au cancer de la prostate et/ou le traitement et/ou la prévention de l'incontinence urinaire.

## Patentansprüche

1. Pharmazeutische Zusammensetzung in Form einer Kapsel, enthaltend eine Füllstoffzusammensetzung, die im Verhältnis zum Gesamtgewicht der Füllstoffzusammensetzung enthält:
- 50 bis 90 Gew.-% eines lipido-sterolischen Extrakts von Serenoa Repens und
- 10 bis 50 Gew.-% zumindest eines Polyethylenglycols mit einem Molekulargewicht zwischen 6 000 und 35 000.

2. Pharmazeutische Zusammensetzung nach Anspruch 1, **dadurch gekennzeichnet, dass** die Füllstoffzusammensetzung 55 bis 60 % lipido-sterolischen Extrakt von Serenoa Repens und 40 bis 45 % an zumindest einem Polyethylenglycol mit einem Molekulargewicht zwischen 6 000 und 35 000 enthält.

3. Pharmazeutische Zusammensetzung nach Anspruch 2, **dadurch gekennzeichnet, dass** die Füllstoffzusammensetzung 58,2 % lipido-sterolischen Extrakt von Serenoa Repens und 41,8 % an zumindest einem Polyethylenglycol mit einem mittleren Molekulargewicht zwischen 6 000 und 35 000 enthält.

4. Pharmazeutische Zusammensetzung nach einem der Ansprüche 1 bis 3, **dadurch gekennzeichnet, dass** das zumindest eine Polyethylenglycol ausgewählt ist aus Polyethylenglycol mit einem mittleren Molekulargewicht von 10 000, Polyethylenglycol mit einem mittleren Molekulargewicht von 20 000 und Polyethylenglycol mit einem mittleren Molekulargewicht von 35 000.

5. Pharmazeutische Zusammensetzung nach einem der Ansprüche 1 bis 4, **dadurch gekennzeichnet, dass** sie 320 mg lipido-sterolischen Extrakt von Serenoa Repens enthält.

6. Pharmazeutische Zusammensetzung nach einem der Ansprüche 1 bis 5, wobei die Kapsel eine Hartmantelkapsel ist.

7. Pharmazeutische Zusammensetzung nach einem der Ansprüche 1 bis 6, **dadurch gekennzeichnet, dass** die Kapsel von der Größe 1,0 oder 0 el, vorzugsweise von der Größe 0 ist.

8. Verfahren zum Herstellen einer pharmazeutischen Zusammensetzung nach einem der Ansprüche 1 bis 7, **dadurch gekennzeichnet, dass** der lipido-sterolische Extrakt von Serenoa Repens mit dem zumindest einen Polyethylenglycol durch gemeinsames Schmelzen vermischt wird und die erhaltene Zusammensetzung durch Abfüllen in Kapseln verpackt wird, wobei die Kapseln dann versiegelt werden.

9. Verfahren nach Anspruch 8, **dadurch gekennzeichnet, dass** die Versiegelung durch Banderolieren oder Aufsprühen eines wässrig-alkoholischen Sprays erfolgt.

10. Pharmazeutische Zusammensetzung nach einem der Ansprüche 1 bis 7 zu deren Verwendung als Arzneimittel.

11. Pharmazeutische Zusammensetzung nach Anspruch 10 zu deren Verwendung bei der Behandlung und/oder Vorbeugung von Störungen der Prostata, vorzugsweise bei der Behandlung und/oder Vorbeugung von gutartiger Prostatahyperplasie und/oder Prostatakrebs und/oder Prostatitis und/oder von damit zusammenhängenden Beschwerden, wie etwa beim Wasserlassen, die mit gutartiger Prostatavergrößerung und/oder Prostatakrebs verbunden sind, und/oder bei der Behandlung und/oder Vorbeugung von Urininkontinenz.

## Claims

1. Pharmaceutical composition in the form of a capsule containing a filling composition comprising, by weight in relation to the total weight of the filling composition:
- 50 to 90% of a Serenoa repens sterol lipid extract, and
- 10 to 50% of at least one polyethylene glycol with an average molecular weight of between 6000 and 35,000.

2. Pharmaceutical composition according to claim 1, **characterised in that** the filling composition comprises 55 to 60% of the Serenoa repens sterol lipid extract and 40 to 45% of the at least one polyethylene glycol with an average molecular weight of between 6000 and 35,000.

3. Pharmaceutical composition according to claim 2, **characterised in that** the filling composition comprises 58.2% of the Serenoa repens sterol lipid extract and 41.8% of the at least one polyethylene glycol with an average molecular weight of between 6000 and 35,000.

4. Pharmaceutical composition according to any of claims 1 to 3, **characterised in that** the at least one polyethylene glycol is chosen from polyethylene glycol with an average molecular weight of 10,000, polyethylene glycol with an average molecular weight of 20,000, and polyethylene glycol with an average molecular weight of 35,000.

5. Pharmaceutical composition according to any of claims 1 to 4, **characterised in that** it comprises 320 mg of the Serenoa repens sterol lipid extract.

6. Pharmaceutical composition according to any of claims 1 to 5, wherein the capsule is a hard-shelled capsule.

7. Pharmaceutical composition according to any of claims 1 to 6, **characterised in that** the capsule size is 1, 0 or 0 el, preferentially size 0.

8. Method for preparing a pharmaceutical composition according to any of claims 1 to 7, **characterised in that** the Serenoa repens sterol lipid extract is mixed with the at least one polyethylene glycol by co-fusion and **in that** the composition obtained is packaged by filling in capsules, said capsules then being sealed.

9. Method according to claim 8, **characterised in that** the sealing is performed by wrapping or spraying with a hydroalcoholic spray.

10. Pharmaceutical composition according to any of claims 1 to 7, for use thereof as a medicinal product.

11. Pharmaceutical composition according to claim 10, for use thereof in the treatment and/or prevention of prostate disorders preferentially in the treatment and/or prevention of benign prostate hyperplasia and/or prostate cancer and/or prostatitis and/or associated disorders such as urinary disorders related to benign prostate hypertrophy and/or prostate cancer and/or the treatment and/or prevention of urinary incontinence.
